# EUROPEAN PATENT SPECIFICATION

(11) **EP 1 560 606 B1**
(45) Date of publication and mention of the grant of the patent: **24.01.2007**
(21) Application number: 03753209.0
(22) Date of filing: 29.10.2003
(51) Int. Cl.: A61L 9/12

(54) **POWERED DISPENSING DEVICES FOR THE DELIVERY OF EVAPORABLE MATERIALS**
ELEKTRISCH BETRIEBENE VORRICHTUNG ZUR ABGABE VON VERDAMPFBAREN STOFFEN
DISTRIBUTEURS A MOTEUR CONCUS POUR DIFFUSER DES MATIERES VAPORISABLES

(30) Priority: 12.11.2002 US 425387 P
(43) Date of publication of application: 10.08.2005
(73) Proprietor: Givaudan SA, 1214 Vernier-Genève (CH)
(72) Inventor: BROWN, Colin, Egham, Surrey TW20 8LP (GB); GOHIL, Kishen, New Malden, Surrey KT3 6HB (GB)
(74) Representative: McStea, John Anthony
(86) International application number: PCT/CH2003/000705
(87) International publication number: WO 2004/043565

(56) References cited:
- EP-A- 0 882 459
- GB-A- 1 454 040
- US-A- 3 633 881
- US-A- 3 804 592
- US-A- 5 114 625
- US-A- 5 788 569
- US-B1- 6 254 065

## Description

The present invention relates to dispensing devices, especially powered dispensing devices, useful for the delivery of a volatile or evaporable material to an ambient environment.

Various devices useful for the delivery into an ambient environment, such as a room or interior of a vehicle, of evaporable materials, such as fragrances, odour masking agents, insecticides and medicaments, are known to the art. Typically, such a device includes a reservoir containing an evaporable material, which reservoir has a neck through which protrudes a wick. The wick transports the liquid by means of capillary action from the reservoir to the ambient environment, into which the liquid evaporates or volatilizes. Such devices are simple, cheap and generally effective. In certain embodiments, they are supplied as part of a larger apparatus, which may provide an external feature for static diffusion systems or it may include a means of diffusing the volatile liquid. Examples of the latter include a heat source, which may surround at least part of the wick, and which, when heated, induces more rapid volatilization of the liquid, or a fan that causes more rapid volatilization of the liquid into the ambient environment.

While such devices have proved useful, they are not free from shortcomings. For example, when a delivery device relies upon a heating element to facilitate the evaporation of a volatile material, the material released is typically driven only by convection currents of the rising hot air released from the device, and the dissemination of the volatile material is dependent on their speed of movement alone. Where such devices are powered by mains electricity, the location of the devices is generally limited to the power outlets available, and the release of the evaporable material thus tends to be largely limited to the vicinity of these outlets. In addition, the operating temperatures of such devices are often high, sometimes between about 65°C and about 140°C, and this can not only degrade chemical materials (evaporable materials, materials of construction of the devices) but may also pose a potential hazard to small children and pets coming into contact with the delivery device.

Battery-powered devices are known to the art, but these are often complicated to manufacture, requiring the assembly of a relatively large number of elements. Such devices usually deliver their fragrance material from a reservoir, said reservoir often taking the form of a porous or absorbent patch, sponge, or gauze, which is placed at the exhaust face of a fan. In operation, such devices typically draw air through the fan, and the exiting air is forced through the reservoir, thereby forcing the release of the vaporizable material to the ambient environment. While such prior art devices may be in some ways effective, the positioning of the reservoir often acts to impede flow of the air being exhausted from the fan. At the same time, the use of an absorbent patch, sponge, or gauze tends to allow the undesirable release of a larger quantity of the vaporizable material earlier in its service life.

US-A-633 881 and US-A-3 804 592 both disclose devices using a fan that cause a flow of air to pass by a wick connected to a reservoir and thence exhausting the air to the atmosphere.

There is a need in the art for improved devices useful for the delivery of evaporable materials.

The present invention provides a powered dispensing as recited in claim 1.

In one aspect, the present invention provides a powered dispensing as recited in claim 1 characterized in that the blower receives said flow air containing evaporable material in a direction that is generally perpendicular to the air-containing evaporable material exhausted from the blower.

In a second aspect of the present invention, there is provided a powered dispensing as recited in claim 1 characterized in that the rotational axis of the blower is perpendicular to the axis of the wick, the blower receives said air containing evaporable material in a first direction which is generally perpendicular to the air containing evaporable material, which is exhausted from the blower in a second direction and into a manifold.

In a third aspect of the present invention, there is provided a powered dispensing device as recited in claim 1 characterized in that it comprises a fan, which, when operating, induces a flow of air from the ambient environment in a direction parallel to that of the air drawn into the fan, through the headspace, and out through the manifold to the atmosphere.

The invention further provides a method of providing an evaporable material to an atmosphere as recited in claim 5.

Provided that the device is arranged so that the embodiments hereinabove described are possible, the size, shape and construction materials of the device are not narrowly critical and a wide variety of possibilities exist. Plastics materials are preferable, as they are cheap, easily formed into complex shapes, and can be attractively coloured. Naturally they must be chosen such that they can withstand any exposure to any solvent in the evaporable liquid. Any other suitable material, such as metals and ceramics, may also be used.

The wick that is used in the dispensing devices according to the present invention to transport the evaporable material from the reservoir to the headspace by means of capillary action may be formed of any suitable material. Such materials include those based on natural or synthetic fibers, woven or non-woven fabrics, cords, rods or other articles, porous media such a those based on celluloses such as cardboard- based and paper-based wicks, graphite and carbonaceous wicks, as well as porous synthetic polymers and porous ceramic materials. Examples of commercially-available materials useful for the manufacture of suitable wicks include porous synthetic polymers from Porex Corp., (Fairburn, GA, USA) and Micropore Plastics Inc. (Stone Mountain, GA) as well as porous ceramics available from Rauschert GmbH & Co.-KG (Germany).

In the preferred embodiments of the powered dispensing device according to the invention, a blower is used, rather than a conventional fan. Blowers can be distinguished from fans by the fact that they deliver air in a direction that is perpendicular to the blower axis, usually at a relatively low air-flow rate, but such blowers are capable of operating against a higher pressure. Fans deliver air in an overall direction which is parallel to the fan blade axis typically at a relatively high air flow rate, but are usually capable of operating only against lower pressures. In each case, the blower axis, or the fan blade axis, is defined as the rotational axis of the motor or of the shaft supporting the respective blower or fan blades. A fan may be used in some embodiments of the invention, but those using a blower are preferred. Suitable blowers and fans of a sufficiently small size are now readily and cheaply available form the industries that supply such devices to the computer industry.

By "manifold" is meant a component, which, when attached to the device, defines a chamber having a plurality of openings. The manifold may be equipped with means for partially or completely closing the openings, so that the emission of evaporable material can be regulated. The manifold is arranged on the device, so that the fan or blower blows air containing evaporable material into the manifold and hence into the atmosphere.

The evaporable material, which may be used in the dispensing devices taught herein, may be any volatile or evaporable material, but is preferably one or more liquids which have a cosmetic effect such as a fragrancing or odour masking effect such as may be based on one or more essential oils, or volatile liquids which have an insecticidal effect or a medical effect. By way of non-limiting example, useful essential oils include one or more of: anethole 20/21 natural, aniseed oil china star, aniseed oil globe brand, balsam (Peru), basil oil (India), black pepper oil, black pepper oleoresin 40/20, Bois de Rose (Brazil) FOB, borneol flakes (China), camphor oil, canaga oil (Java), cardamom oil, cassia oil (China), cedarwood oil (China), cinnamon bark oil, cinnamon leaf oil, citronella oil, clove bud oil, clove leaf, coriander (Russia), coumarin (China), cyclamen aldehyde, diphenyl oxide, ethyl vanillin, eucalyptol, eucalyptus oil, eucalyptus citriodoura, fennel oil, geranium oil, ginger oil, ginger oleoresin (India), white grapefruit oil, guaiacwood oil, gurjun balsam, heliotropin, isobomyl acetate, isolongifolene, juniper berry oil, L-methyl acetate, lavender oil, lemon oil, lemongrass oil, lime oil, litsea cubeba oil, longifolene, menthol, methyl cedryl ketone, methyl chavicol, methyl salicylate, musk ambrette, musk ketone, musk xylol, nutmeg oil, orange oil, patchouli oil, peppermint oil, phenyl ethyl alcohol, pimento berry oil, pimento leaf oil, rosalin, sandalwood oil, sandenol, sage oil, clary sage, sassafras oil, spearmint oil, spike lavender, tagetes, tea tree oil, vanilin, vetyver oil (Java), wintergreen.

These and other suitable materials which may be useful in the volatile liquid may be commercially obtained from a variety of suppliers including: Givaudan Corp. (Teaneck, NJ); Berje Inc. (Bloomfield, NJ); BBA Aroma Chemical Div. of Union Camp Corp. (Wayne, NJ); Firmenich Inc. (Plainsboro NJ); Quest International Fragrances Inc. (Mt. Olive Township, NJ); Robertet Fragrances Inc. (Oakland, NJ), or from other suppliers not necessarily listed herein.

The volatile liquids may be provided in the form of neat compositions, or may be provides as aqueous mixtures, organic mixtures or aqueous-organic mixtures which include of one or more volatile or evaporable materials.

The invention is now further described with reference to the accompanying drawings, which depict preferred embodiments of the invention and which are not limiting in any way on the scope thereof.

Figure 1 depicts a longitudinal part-cross-sectional view of a preferred powered dispensing device according to the invention.

Figure 2 depicts an exploded view of the powered dispensing device according to Figure 1.

Figure 3 depicts a transverse cross-section along the line XX' of the embodiment of Figure 1.

Figure 4 depicts a longitudinal part-cross-section of another embodiment of the invention.

Figure 5A depicts a transverse cross-section along the line YY' of Figure 4.

Figure 5B depicts an alternative embodiment of Figure 5A.

Figure 6 depicts a longitudinal part-cross-sectional view of a third embodiment of the invention.

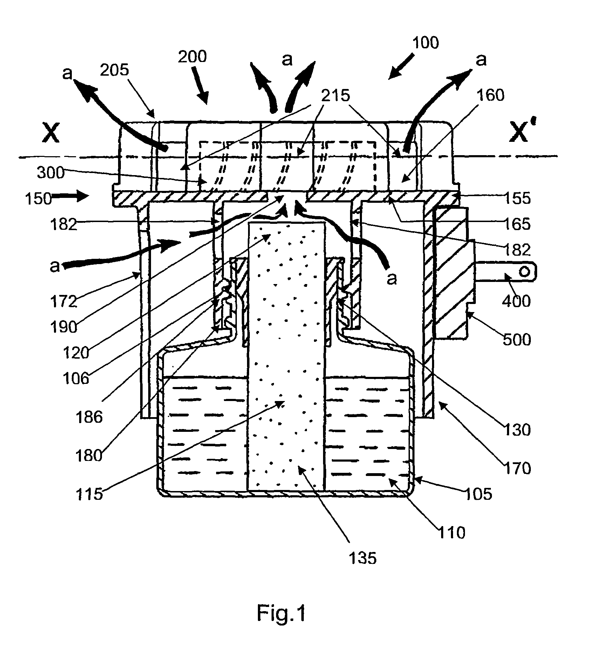

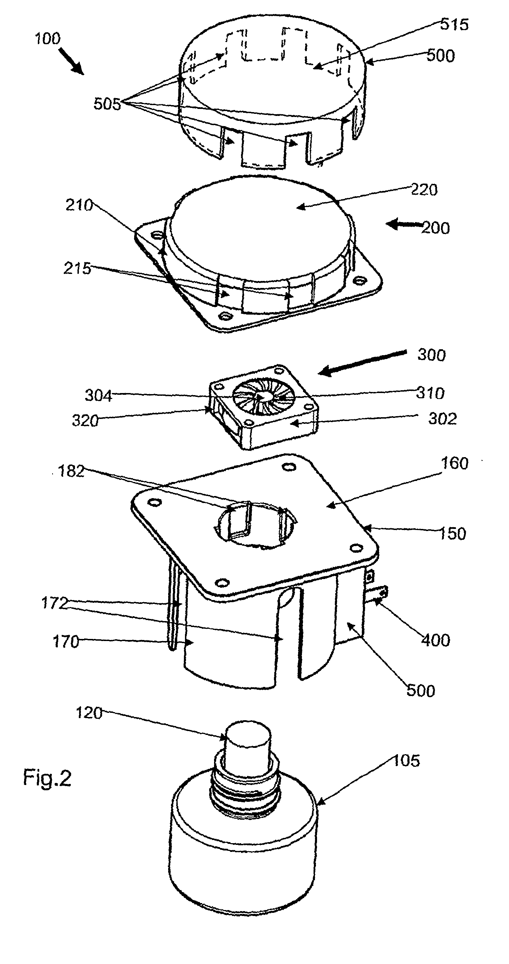

In the following description of the embodiment of Figure 1, reference should also be made to Figure 2, in which some of the features may be more clearly seen. A powered dispensing device, generally indicated as 100, comprises a reservoir 105 containing evaporable liquid 110. The reservoir has a neck 130, through which is inserted a wick 115, such that the lower end of the wick 135 is immersed in the liquid 110 and the upper end 120 protrudes from the neck. The wick is held in place in the neck by an insert 125 that fits tightly into the neck and is a close fit around the wick.

The reservoir and wick fit into a body portion generally indicated as 150. The body portion has the form of a flat plate 155 having an upper surface 160 and a lower surface 165, with reference to the reservoir in its normal operating position, from which lower surface 165 depend two concentric annular skirts, an outer skirt 170 and an inner skirt 180. The inner skirt is equipped with screw threads 186 on its inner surface, which screw threads are adapted to mate with corresponding screw threads 106 on the neck 130 of the reservoir, such that the reservoir can be screwed into the body portion and such that the upper end of the wick 120 is in an headspace defined by the plate 155 and the inner skirt 180. The plate 150 has an orifice 190, positioned above the upper end 120 of the wick.

The outer skirt 170 is dimensioned so that its diameter exceeds that of the reservoir 170, and so that it extends lower than the neck 130 and part-way down the body of the reservoir 105, concealing part of the reservoir but allowing for easy unscrewing when necessary.

Both skirts comprise a plurality of openings that allow air to reach the wick 120. These are 172 in outer skirt 170 and 182 in inner skirt 180.

On the upper surface 160 of the plate 150 is mounted a manifold assembly generally indicated as 200 (shown unsectioned in Figures 1, 4 and 6)). This comprises a cover 205 having a sidewall 210, the sidewall having a plurality of openings 215. Also mounted on this upper surface and within the manifold is a blower 300 (shown schematically in outline in Figures 1, 4 and 6). This is typically of the type used to cool laptop computers, having a series of vanes, which rotates in a horizontal plane with respect to the normal orientation of the device when in use. It is powered by mains electricity, which is supplied by means of a conventional plug 400 of the type that can be inserted into the wall socket. The electricity supply is additionally supplied with the necessary electrical circuitry that allows the device to function, for example a transformer or rectifier. Other possibilities include timers, optical switches, mechanical switches, light and sound sensors, proximity sensors, thermocouples, proximity sensors, sound sensors, thermocouples, "X-10" circuitry that responds to a control signal passed along electrical circuit of a building or a vehicle. These are all generally indicated as 500

In operation, evaporable material 110 is drawn via capillary action up the wick 115 to the upper end 120, after which it evaporates into the headspace of the body portion 150, and also possibly into the region between the inner skirt 180 and the outer skirt 170 of the body portion 150 and possibly even into the ambient environment. Once the blower 300 is energized, the blower receives air containing evaporable material in a first direction, here in a direction parallel to its rotational axis via the orifice 190, this air entering through openings 172 in outer skirt 170 and openings 182 in inner skirt 180. Thereafter, the air containing evaporable material is exhausted from the blower 300 in a second direction that is generally perpendicular to the first direction, in this case, in a plane approximately parallel to the plane of rotation of the blower 300. This is made possible by the presence of one or more peripheral openings present within the blower 300. Thereafter, the air containing evaporable material leaves the manifold assembly 200 via the openings 215. The flow of air through the powered dispensing device 100 is depicted by the small arrows labeled "a".

The exploded view of Figure 2 shows the various components of the embodiment of Figure 1. The plurality of openings 172 in the outer skirt 170 may be seen. Similarly, the inner skirt 180 also includes a number of openings 182 which permit the passage of air from the interior of the body part 150, and into the proximity of the upper end 120 of the wick when the powered dispensing device 100 is assembled.

The blower 300 consists of a housing 302 surrounding a series of vanes 310 mounted on a hub 304 and driven by a motor. The housing 302 comprises at least one open sidewall portion 320 that permits the passage of air therethrough when the blower 300 operates (it is possible to have more than one open sidewall portion). When the manifold assembly 200 is placed on the plate 150 over the blower 300, the top surface 220 of the manifold assembly 200 covers the blower 300 so that air exiting the blower 300 passes out only through the open sidewall portion 320.

The embodiment shown in Figure 2 additionally comprises a sliding cover 500, essentially a flat, hollow cylinder open at one end and comprising a sidewall 510, an end portion 515, and in the sidewall a plurality of openings 505. The sliding cover 500 is dimensioned to fit tightly and rotatably over the manifold assembly 200. In use, the sliding cover 500 may be rotated so as to cover or uncover one or more of the openings 215 of the manifold assembly 200.

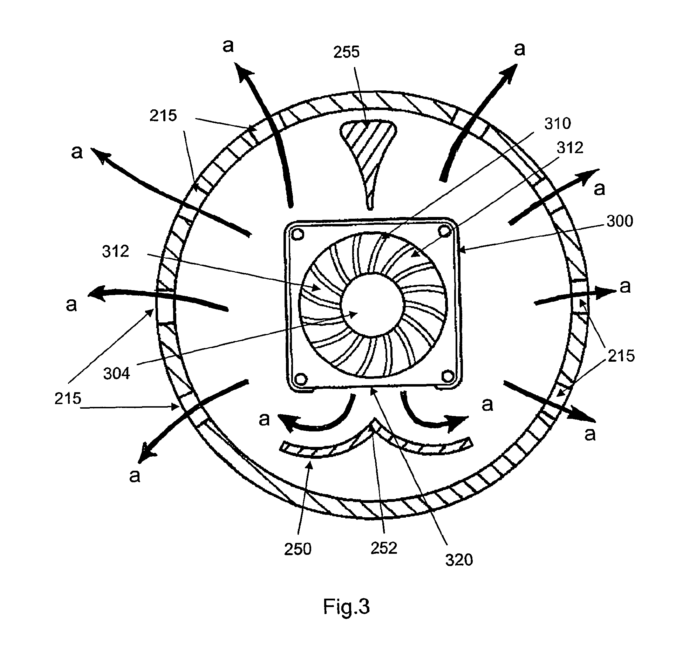

In the cross-section view Figure 3, the interior of the manifold may be seen, looking down on the blower from above. Depicted is the blower 300 having one open sidewall 320. The blower 300 comprises an intake port 312 generally visible as a circle within which is seen the hub 304 and vanes 310. Depending from the top surface 220 of the manifold assembly 200 are baffles 250 and 255. Baffle 250 has the form of two arcuate sections joined at one end to form in plan a broad arrowhead, the point of the arrow 252 being directed towards and located centrally to the open sidewall 320 of the blower. As can be seen by the arrows labeled "a" which are intended to indicate the direction of airflow within the manifold assembly 200, air exiting the blower 300 via the open sidewall 320 impacts upon the baffle 250 and is directed to opposite sides of the manifold assembly 200, and along opposite sides of the blower 300. This directed air, which contains evaporable material, may then exit the manifold assembly 200 through one or more of the openings 215. The second baffle 255 is positioned within the manifold assembly 200 in a position diametrically opposite to that of the first baffle 250. This baffle 255 has, in plan, a generally triangular cross section, and it is intended to direct the air containing evaporable material within the interior of the manifold assembly 200 to one or more openings 215, and thus into the ambient environment. It is naturally possible to vary the number and shape of baffles; such modifications are well within the skill of the art.

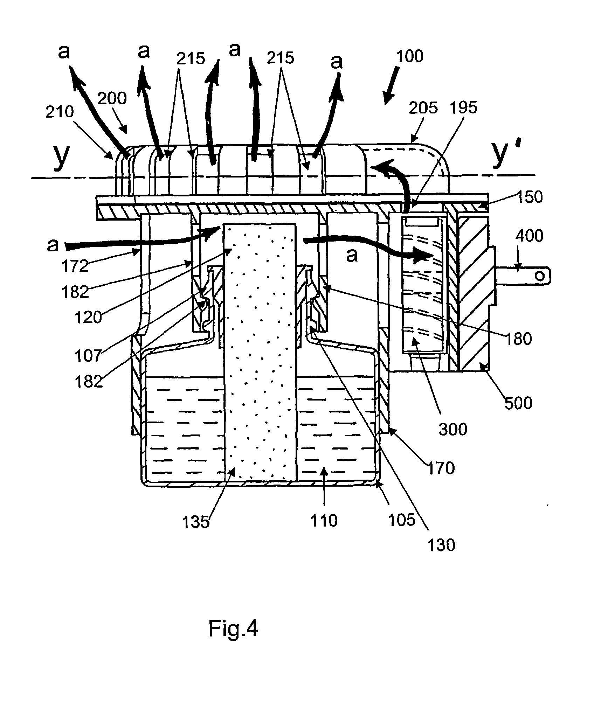

The embodiment of Figure 4 is essentially the same as that of Figure 1, but the blower 300 is mounted underneath the plate 150 and adjacent to it, but outside the outer skirt 170 and orientated such that the axis of rotation is perpendicular to that of the longitudinal axis of the wick. The reservoir is fitted to the inner skirt 180 by means of a snap-fit attachment means, formed of corresponding elements 107 and 186 in the neck 130 and skirt 180 respectively.

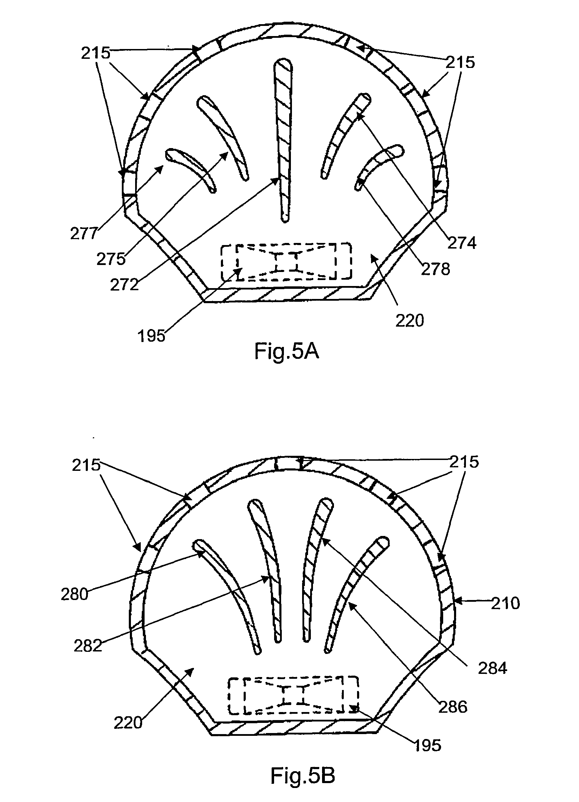

In this case, the central orifice 190 of Figure 1 is replaced by an orifice 195 in the plate 150 (see also Figures 5A, 5B for its shape and location), corresponding with an open sidewall portion in the blower housing, of the type depicted in Figure 2. In operation, the blower draws air containing evaporable material from the headspace surrounding the upper end of the wick 120, through orifices in the inner and outer skirts and blows it through orifice 195 into the manifold assembly 200. The air containing the evaporable material passes into the blower in a first direction generally parallel to the rotational axis of the blower 300, and exits the blower in a second direction generally perpendicular to the rotational axis of the blower 300. Thereafter, the air containing the evaporable material exits the manifold through one or more of the openings 215 and into the ambient environment, in the direction of the arrows a. The manifold assembly may be equipped with one or more baffles, to assist in distributing the air.

Figures 5A and 5B depict two possible alternative baffle arrangements within the manifold of an embodiment according to Figure 4. Figure 5A shows an interior with a series of baffles, 272, 274, 275, 277, 278. Baffle 272 is generally straight and is generally perpendicular to the orifice 195. The paired baffles 274, 275 are approximately one-half as long as baffle 272, and are angled with respect thereto but do not contact baffle 272. While the offset angle from baffle 272 may vary, here it is depicted to be approximately 30 degrees from the baffle 272. In a similar manner, paired baffles 277 and 278 are also shorter, again about one-half of the length of baffle 272 and are angled with respect thereto but do not contact baffle 272. Again, while the offset angles may vary, the offset angle between each of baffles 277 and 278 from baffle 272 is approximately 45 degrees. In operation, the baffles 272, 274, 275, 277, 278 deflect the flow of air containing the evaporable substance which enters the manifold assembly 2000 via the orifice 195 (shown in dotted outline), past the baffles and then through one or more of the openings 215.

In Figure 5B, there is a series of baffles, 280, 282, 284, and 286. Each of the baffles is arcuate, and they extend from the orifice 195 towards the sidewall 210. In operation, the baffles 280, 282, 284, and 286 deflect the flow of air containing the evaporable substance which enters into the manifold assembly 2000 via the orifice 195, past these baffles and thereafter through one or more of the openings 215 and into the ambient environment.

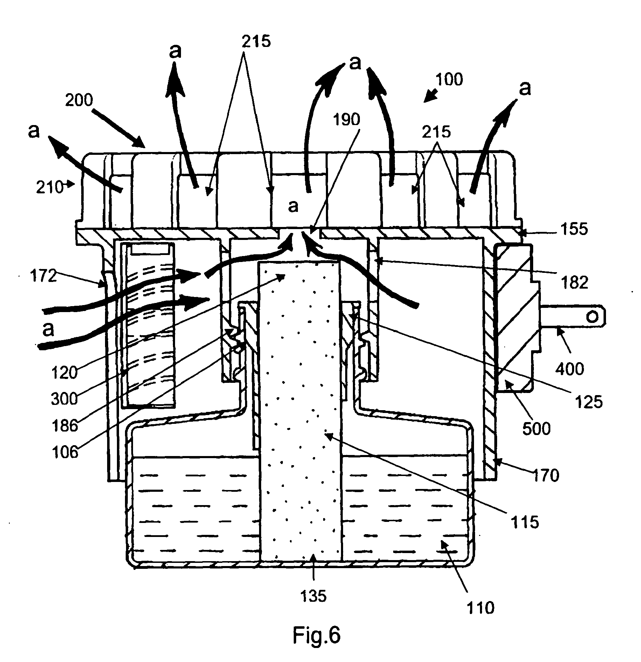

In Figure 6 is depicted an apparatus that is similar in configuration to that of Figure 1. The difference here is that the apparatus uses a fan 300 designed to blow air in a direction parallel to the axis of rotation, rather than a blower, this fan being installed in the space between the inner and outer skirts and is mounted with its rotational axis perpendicular to the longitudinal axis of the wick, in the manner of the blower of Figure 2. This fan draws in air through openings 172 in the outer skirt 170 and exhausts it in the same general direction as the incoming air, through orifices 182 in the inner skirt 180 to the upper end of the wick 120 and the headspace surrounding it. The air containing evaporable material from the headspace leaves the headspace through orifice 190 positioned above the upper end of the wick 120 and enters the manifold assembly 200, exiting therefrom into the atmosphere through the openings 215 in the manifold, along the directions indicated by the arrows a. The manifold may be equipped internally with baffles of the type shown in Figures 3, 5A and 5B.

The embodiments hereinabove described are given by way of example only and the skilled person will readily be able to envisage many variations that fall within the scope of this invention. Some examples:
- The device need not be powered by mains electricity, but by alternative, self-contained energy sources, for example, solar cells or batteries. The modifications to include such possibilites are straightforward.
- The fans in the embodiments shown are adapted to suck air containing evaporable material from a headspace around a wick and blow it into a manifold. The fan can be easily rearranged to suck in air from the atmosphere and blow it into the headspace and thence into the manifold.

## Claims

1. A powered dispensing device (100) adapted to dispense an evaporable material (110) into an atmosphere, comprising a reservoir (105) containing evaporable material (110), a wick (135) extending from said reservoir and providing said evaporable material to a headspace surrounding an exposed end of the wick, and a blower or fan (300), which, when operating, induces a flow of air to pass through the headspace, and convey evaporable material into the atmosphere, the device comprising a body portion (150) in the form of a flat plate (155) having an upper surface (160) comprising an orifice (190,195) and forming a chamber together with a manifold (200) mounted thereon, the manifold comprising a cover (205) having a side wall (210), the sidewall having a plurality of openings (215), the manifold also comprising a plurality of internal baffles for deflecting the air containing evaporable material entering the manifold via the orifice (190,195) past the baffles and leaving the manifold through the openings (215) into the ambient environment.

2. A device according to claim 1, wherein the blower (300) is so arranged that, in operation, it induces a flow of air containing evaporable material (110) from the headspace to pass through the blower, and out into the atmosphere through the manifold openings (215), the blower receiving said flow air containing evaporable material in a direction that is perpendicular to the air containing evaporable material exhausted from the blower.

3. A device according to claim 1, in which the rotational axis of the blower (300) is perpendicular to the longitudinal axis of the wick (135), the blower being arranged such that it receives air containing evaporable material (110) from the headspace in a first direction that is perpendicular to a second direction in which the air containing evaporable material (110) is exhausted from the blower (300) into the manifold (200) and thence into the atmosphere.

4. A device according to claim 1, in which the device comprises a fan that induces a flow of air from the ambient environment in a direction parallel to that of the air drawn into the fan, through the headspace, and out into the atmosphere through the manifold openings.

5. A method of providing an evaporable material to an atmosphere by using a device of one of the claims 1-4.

6. A method according to claim 5, in which the flow of air is caused by a blower (300) and the blower receives a flow of air containing evaporable material in a direction that is perpendicular to the air containing evaporable material exhausted from the blower.

## Patentansprüche

1. Angetriebenes Dispensergerät (100) zur Abgabe eines verdampfbaren Materials (110) in eine Atmosphäre, umfassend ein Reservoir (105), das ein verdampfbares Material (110) enthält, einen Docht (135), der sich von dem Reservoir erstreckt und das verdampfbare Material in einen Kopfraum führt, der ein freiliegendes Ende des Dochts umgibt, und ein Gebläse oder einen Ventilator (300), das bzw. der im Betrieb eine Luftströmung veranlasst, durch den Kopfraum zu strömen und verdampfbares Material in die Atmosphäre zu transportieren, wobei das Gerät einen Körper (150) in Form einer flachen Platte (155) umfasst, die eine Oberseite (160) mit einer Öffnung (190,195) hat und zusammen mit einem darauf montierten Verteiler (200) eine Kammer bildet, wobei der Verteiler einen Deckel (205) mit einer Seitenwand (210) umfasst, die mehrere Öffnungen (215) hat, und der Verteiler auch mehrere innere Prallplatten zum Ablenken der verdampfbares Material enthaltenden Luft aufweist, die durch die Öffnung (190,195) an den Prallplatten vorbei in den Verteiler eintritt und diesen durch die Öffnungen (215) in die äußere Umgebung verlässt.

2. Gerät nach Anspruch 1, bei dem das Gebläse (300) so angeordnet ist, dass es im Betrieb eine verdampfbares Material (110) enthaltende Luftströmung aus dem Kopfraum veranlasst, durch das Gebläse und nach außen durch die Verteileröffnungen (215) in die Atmosphäre zu strömen, wobei das Gebläse die verdampfbares Material enthaltende Luftströmung in einer Richtung entgegennimmt, die senkrecht zu der verdampfbares Material enthaltenden Luft ist, die vom Gebläse abgegeben wird.

3. Gerät nach Anspruch 1, bei dem die Drehachse des Gebläses (300) senkrecht zur Längsachse des Dochts (135) ist, das Gebläse so angeordnet ist, dass es verdampfbares Material (110) enthaltene Luft aus dem Kopfraum in einer ersten Richtung entgegennimmt, die senkrecht zu einer zweiten Richtung ist, in der die verdampfbares Material (110) enthaltende Luft von dem Gebläse (300) in den Verteiler (200) und dann in die Atmosphäre abgegeben wird.

4. Gerät nach Anspruch 1, bei dem das Gerät einen Ventilator enthält, der eine Luftströmung aus der äußeren Umgebung in einer Richtung parallel zu der der vom Ventilator angesaugten Luft durch den Kopfraum und nach außen in die Atmosphäre durch die Verteileröffnungen hervorruft.

5. Verfahren zum Abgeben eines verdampfbaren Materials in eine Atmosphäre durch Verwendung eines Geräts nach einem der Ansprüche 1 bis 4.

6. Verfahren nach Anspruch 5, bei dem die Luftströmung von einem Gebläse (300) hervorgerufen wird und das Gebläse eine verdampfbares Material enthaltende Luftströmung in einer Richtung entgegennimmt, die senkrecht zu der verdampfbares Material enthaltenden Luft ist, die von dem Gebläse abgegeben wird.

## Revendications

1. Dispositif de distribution motorisé (100) agencé pour distribuer un matériau évaporable (110) dans une atmosphère, comprenant un réservoir (105) contenant un matériau évaporable (110), une mèche (135) s'étendant dudit réservoir et fournissant ledit matériau évaporable à un collecteur entourant une extrémité exposée de la mèche et une soufflerie ou ventilateur (300) qui lorsqu'il est en fonctionnement induit un écoulement d'air traversant le collecteur et acheminant le matériau évaporable dans l'atmosphère, le dispositif comprenant une partie de corps (150) sous la forme d'une plaque plane (155) ayant une surface supérieure (160) comprenant un orifice (190, 195) et formant une chambre conjointement avec une tubulure (200) montée sur celle-ci, la tubulure comprenant un couvercle (205) ayant une paroi latérale (210), la paroi latérale ayant une pluralité d'ouvertures (215), la tubulure comprenant également une pluralité de chicanes internes pour dévier l'air contenant le matériau évaporable pénétrant dans la tubulure par l'intermédiaire de l'orifice (190, 195) au-delà des chicanes et quittant la tubulure au travers des ouvertures (215) dans l'environnement ambiant.

2. Dispositif selon la revendication 1, dans lequel la soufflerie (300) est agencée de sorte qu'en fonctionnement elle induit un écoulement d'air contenant le matériau évaporable (110) à partir du collecteur pour traverser la soufflerie et à l'extérieur dans l'atmosphère au travers des ouvertures (215) de la tubulure, la soufflerie recevant ledit écoulement d'air contenant le matériau évaporable dans une direction qui est perpendiculaire à l'air contenant le matériau évaporable dégagé de la soufflerie.

3. Dispositif selon la revendication 1, dans lequel l'axe de rotation de la soufflerie (300) est perpendiculaire à l'axe longitudinal de la mèche (135), la soufflerie étant agencée afin qu'elle reçoive l'air contenant le matériau évaporable (110) du collecteur dans une première direction qui est perpendiculaire à une deuxième direction dans laquelle l'air contenant le matériau évaporable (110) est dégagé de la soufflerie (300) dans la tubulure (200) et par conséquent dans l'atmosphère.

4. Dispositif selon la revendication 1, dans lequel le dispositif comprend un ventilateur qui induit un écoulement d'air à partir de l'environnement ambiant dans une direction parallèle à celle de l'air aspiré dans le ventilateur au travers du collecteur et à l'extérieur dans l'atmosphère au travers des ouvertures de la tubulure.

5. Procédé pour fournir un matériau évaporable à une atmosphère en utilisant un dispositif selon l'une quelconque des revendications 1 à 4.

6. Procédé selon la revendication 5, dans lequel l'écoulement d'air est provoqué par une soufflerie (300) et la soufflerie reçoit un écoulement d'air contenant un matériau évaporable dans une direction qui est perpendiculaire à l'air contenant le matériau évaporable dégagé de la soufflerie.
